# EUROPEAN PATENT SPECIFICATION

(11) **EP 1 049 480 B1**
(45) Date of publication and mention of the grant of the patent: **11.09.2002**
(21) Application number: 99902706.3
(22) Date of filing: 29.01.1999
(51) Int. Cl.: A61K 35/78

(54) **HERBAL ANTI-VIRAL AGENT**
PFLANZLICHES ANTIVIRALES MITTEL
AGENT ANTIVIRAL D'ORIGINE VEGETALE

(30) Priority: 30.01.1998 US 16121
(43) Date of publication of application: 08.11.2000
(73) Proprietor: Rhinopharma AS, 1086 Oslo (NO)
(72) Inventor: PREUS, Hans, Ragnar, N-1440 Drobak (NO)
(74) Representative: Dzieglewska, Hanna Eva
(86) International application number: GB9900316
(87) International publication number: WO99038522

(56) References cited:
- WO-A-96/05849
- DE-A- 2 659 462
- DATABASE WPI Section Ch, Week 9241 Derwent Publications Ltd., London, GB; Class A96, AN 92-334322 XP002103450 & HU 60 126 A (DAVID A), 28 August 1992

## Description

The present invention relates to the use of a composition comprising myrrh and rhatany as the sole active ingredients in the prevention and treatment of viral infections in human and non-human animals. In particular it relates to the use of a myrrh and rhatany composition as a prophylactic and therapeutic of the common cold in human beings.

Myrrh is an aromatic, gummy substance exuded by particular trees and shrubs, principally of the Burseraceae family, which grow in Africa and the Middle East. Most commonly, myrrh is collected as a resin produced by the stem of the gum myrrh tree Commiphora myrrha, Commiphora abyssinica or Balsamodendron myrrh.

Myrrh has been used by mankind for a multitude of applications for thousands of years. It is known to have been a component of embalming mixtures used by ancient Egyptians and has since found a role as an active ingredient in a diverse range of products including insect repellant, treatments for bruises, sprains and flatulence. Its antiseptic and astringent properties have made it popular in remedies for treating wounds, sore teeth and gums and chest infections.

Rhatany is a gum resin extracted from the tissues but particularly the root of plants of the genus Krameria, in particular the species K. triandra and K. argenta. Rhatany is a complex mixture which may comprise neolignans and norneolignans, oligomeric proanthocyanides, tannins, monomeric and oligomeric flavenols and low molecular weight phenols and katechins. Like myrrh, rhatany has found many uses throughout history on account of its anti-bacterial, anti-fungal and anti-hypertensive properties. In particular, it has been used, *inter alia,* as a food preservative, breath freshener (in mouthwash) an astringent and in remedies for the treatment of fungal infections of the skin.

Viral infections are arguably the most recalcitrant of all diseases of infectious aetiology to combat in clinical medicine. Although several prophylactic programs against viral infections have been very successful for example, vaccination against polio, small pox and to a lesser degree influenza, there are very few success stories in the development of actual curative therapies or antiviral antibiotics. In fact, the development of nucleoside analogues such as acycloguanosine which are effective in the control of Herpes simplex I and II infections, is almost the only known effective antiviral agent.

Many common infections, in particular contagious infections of the respiratory tract are of viral aetiology, such as the common cold, which is caused principally by Rhinoviruses, Coronaviruses and respiratory syncytial virus (RSV). Taxonomically, Rhinoviruses and Coronaviruses are members of the Picornaviridae which are small, single stranded RNA viruses. Rhinoviruses, which cause around 80% of cases of the common cold are known to exist in over a hundred different serotypes and are subject to rapid genomic mutation, a phenomenon called antigenic drift. This rapid and unpredictable "evolution" results in a lack of constancy in antigenic determinants and thus accounts, at least in part, for the inability to develop effective vaccines against the common cold and to date, symptomatic therapy remains the mainstay of common cold therapy.

Vast amounts of scientific and economic investment have been made in trying to establish safe and effective remedies and prophylactics for viral infections with very little success. The fact that many viral infections are very common, notably the common cold which, although seldom a serious illness, causes much suffering and economic loss in terms of inability to work, means that there is a very great need for safe, effective and relatively inexpensive prophylactics and therapies or remedies for viral infections.

Surprisingly therefore, the Applicant has shown that a composition comprising myrrh and rhatany as the sole active agents, is effective in preventing viral infections, or in treating or alleviating the symptoms of viral infections.

This finding is remarkable since myrrh and rhatany extracts have, as indicated previously, been used extensively by mankind for such a long period, and although they are each known to possess antiseptic and astringent properties, no hint or suggestion has ever been made that a combination comprising these extracts might be useful in the treatment and/or prevention of viral infections or disease in humans or other mammals.

According to one aspect therefore, the present invention provides the use of myrrh and rhatany as the sole active ingredients in the preparation of a medicament for the prevention and/or the treatment of viral infections or diseases in a human or non-human animal subject.

The invention also provides a composition comprising myrrh and rhatany as the sole active ingredients together with at least one pharmaceutically acceptable carrier or excipient for use in the prevention and/or treatment of viral infections or viral diseases in a human or non-human animal subject.

The invention further provides the use of an alcoholic or hydro-alcoholic solution of myrrh and rhatany in the manufacture of a medicament for the prevention and/or treatment of viral infections or viral diseases in a human or non-human animal subject.

By "sole active agent" as used herein is meant that the myrrh and rhatany are included in the composition or products of the invention, or used in the methods of the invention as the sole agents active in combatting the viral infection or disease. The term "combatting" as used herein includes both prophylaxis and therapy, ie. both preventing and treating viral infections or diseases, including relief or alleviation of symptoms, or a halting or slow-down in the progression or development of the disease or infection. The term "anti-viral" includes all forms of action against the virus, including an activity in preventing or reducing infection by the virus. or viral transfer in the host, viral survival or replication etc.

Both myrrh and rhatany exist or are available in many different forms, for example as resins, tinctures or other solutions, powders, dried plants or parts of plants, in particular the bark, stem or root of the plant as appropriate, all of which are suitable for use in the present invention. Appropriate sources or preparations of myrrh or rhatany are widely described in the literature or are commercially available. Preparations of rhatany are for example known in the art under the name of rhatania or rhatania extract.

Extracts of myrrh and rhatany from the appropriate plant parts (e.g. myrrh bark or rhatany/rhatanhia root) may be prepared using solvents such as water, ethanol, methanol, hexane, ether, acetone, ethyl acetate, toluene, benzene, propylene glycol, glycerin and the like or a mixture thereof. Depending on the condition of the extract fraction or preparation, a portion of the extract fraction may, for example, be separated from the solvent by evaporation and then directly utilized as an extract having antiviral properties, or some extract fraction may be further extracted again with the aforementioned solvent for the purpose of increasing the content of such effective ingredient and the secondary extract fraction thus obtained may be separated from the solvent, for example, by evaporation to give an extract having antiviral properties.

Derivatives or analogues of myrrh and rhatany may also be used in the present invention and are subsumed within the general terms 'myrrh' and 'rhatany'. A derivative of rhatany, ratanhiaphenol may be obtained, for example, by extracting ratanhia or rhatany extract as mentioned above, with a mixture of dilute hydrochloric acid and ethyl ether, evaporating the ether layer, then dissolving the residue in chloroform, fractionating the solution on silica gel (74 to 149µ) with chloroform and hexane 7:3 eluants to collect the Ratanhiaphenol fraction, and further recrystallizing from petroleum ether. The thus obtained ratanhiaphenol is in the form of colourless needle-like crystals.

Any of the processes described in the prior art for obtaining extracts or tinctures of myrrh or rhatany/ratanhia or ratanhiaphenol can be used to carry out the present invention, for example as described in US Patent No. 4,886,667. Tinctures (ie. alcoholic or hydro-alcoholic solutions) of myrrh and rhatany are commercial products (which may be purchased for example from NAF, Oslo, Norway) which may conveniently be used according to the invention.

The amount of myrrh or rhatany extract (in whatever form) which may be used in compositions according to the invention ranges from 0.00005 to 25 w/w%, and preferably from 0.005 to 20 w/w% depending on the method of administration. A tincture may be prepared for example, by dissolving 20g of myrrh or rhatany powder in 100g of alcohol. This may be used undiluted for topical application or may be diluted one part in 25 with water (for example 4 ml of stock solution into 96 ml of water) for use as a mouthwash or gargle. The resins, tinctures and other formulations of these natural extracts have all been shown through use to be safe when taken internally and applied. Thus, the myrrh and rhatany may be formulated in any manner known in the medicinal arts for use in the invention. In addition to myrrh and rhatany it will be understood that derivatives and analogues of both of these active agents, whether naturally or chemically/synthetically derived, as well as partially purified or fractionated preparations, are within the scope of the present invention. Preferably however, the myrrh and rhatany of the present invention are natural extracts.

The active ingredients may be incorporated, with one or more conventional carriers, solvents, diluents and/or excipients, to produce conventional preparations such as tablets, pills, powders, lozenges, sachets, cachets, elixirs, suspensions, emulsions, solutions, syrups, aerosols (as a solid or in a liquid medium), ointments, soft and hard gelatin capsules, preparations for topical application sterile packaged powders, mouthwash or mouth rinse and the like.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, ethanol, water/glycol, water/polyethylene, glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The medicament may be formulated so as to provide quick, sustained or delayed release of the active ingredients after administration to the patient by employing procedures well known in the art.

In a preferred embodiment, the active ingredients in the form of tinctures are mixed together with water and the resulting solution simply taken orally. Diluted tinctures of myrrh and rhatany may also of course be taken separately ie. without admixture.

Alternatively, the myrrh and rhatany components of the present invention are in the form of granulated powder, or as in a more preferred embodiment of the present invention, as a tincture comprising an extract of myrrh or rhatany resin in, for example, between 45% and 96% alcohol.

Preferably, the herbal ingredient tincture of myrrh is obtained by extracting myrrh from the gum resin of trees and shrubs of the genus Commiphora, in particular Commiphora myrrha, Commiphora abyssinica or the tree Balsamodendron myrrh, using concentrated, for example 96%, conventional alcohol such as ethanol. The herbal ingredient tincture of rhatany is obtained in a manner analagous to that described above for myrrh, from the gum resin of Krameria triandra or Krameria argenta root, using concentrated, for example 96% conventional alcohol such as ethanol.

Such extracts or myrrh and rhatany obtained using, for example, 96% ethanol, may then be either diluted using water to the required concentration of alcohol or required concentration of active ingredient as desired. Alternatively, the alcoholic tinctures may be extracted again using concentrated, for example 96% or less concentrated alcohol, for example between 50 and 90% alcohol, and the resultant tinctures diluted with water to the desired concentration of alcohol or desired concentration of active ingredient as appropriate.

Generally, commercially sold tinctures will vary in their final alcohol and myrrh or rhatany composition or concentration depending on the different brand sold or country of marketing. Typically, such a tincture of myrrh may be marketed at a final alcohol concentration of between 50 and 90% alcohol and a tincture of rhatany may be marketed at a final alcohol concentration of between 50% and 75% alcohol and between. It should be appreciated however that tinctures of any concentration of alcohol and myrrh or rhatany are suitable for utilisation in the present invention and that such tinctures may be diluted and/or mixed as appropriate.

Thereafter, the tincture of myrrh and tincture of rhatany are combined together to form a mixture. Preferably, the tincture of myrrh and tincture of rhatany are combined in equal amounts of 50% each component to form the mixture. It is understood that the percent of each herbal component in the mixture may vary depending on the type of disease and method of treatment. Therefore, each herbal component of the present invention may be present in the mixture in an amount of from about 1% to about 99%, by weight, of the component.

As an example of preparing a composition for use as a mouthwash, a tincture of myrrh may be prepared by mixing 20g of myrrh with 7.5g of distilled water and 92.5g of 96% alcohol and allowing the active ingredient to be extracted from the myrrh powder into the liquid phase forming a tincture. The resultant tincture from such a process comprises a final alcohol concentration of about 84%. A rhatany tincture may be prepared analagously but preferably to a final alcohol concentration of about 64%. 2 to 3ml of each tincture may then be added to 100ml of water for utilisation as a mouthwash.

After the herbal components are combined to form a mixture, the mixture may be diluted in a solvent to form a medicament ready for use. It is understood that the solvent may vary depending on the particular disease and method of treatment with which the composition is to be utilized. For example, if the herbal composition of the present invention is used as a mouth rinse or gargle for treating, for example, the common cold or other viral infections of the mouth, throat or respiratory tract, the herbal mixture is diluted in a solvent such as tap or distilled water. Preferably, for forming a mouth rinse the mixture of the present invention is diluted in tap water in an amount from about 1:10 to about 1:1000. For purposes of illustration the herbal composition may be used in the prophylactic treatment of such above-mentioned conditions by mixing about a 1:25 dilution, by weight, of a mixture of rhatany and myrrh tinctures with tap water. The resultant solution may be used as a mouth rinse.

The prescribed dosage will vary, for example, one may rinse once daily for a period of about one minute per rinse but this, if necessary, may be repeated after 1-2 hours. It is within the scope of the present invention to add a conventional dye or other suitable additive to colour and/or add taste to the rinse that does not change the efficacy of the herbal composition.

The mixture of the present invention comprising tincture of rhatany and tincture of myrrh is preferably diluted 1:25, by weight, with tap water. Then, at first notice of, for example, a developing sore throat or other symptom of the common cold, an effective regimen of treatment may consist of rinsing once or twice daily for about thirty seconds each per rinse.

Vitamins, minerals or other additives such as zinc may optionally be incorporated or added to the mixture.

The active ingredients are not necessarily co-administered in a single formulation and the myrrh and rhatany may optionally be administered to or taken by the patient separately, sequentially or simultaneously. The formulations in which the myrrh and rhatany are prepared may be the same or different and the route of administration of each formulation may likewise be the same or different.

Accordingly, a further aspect of the invention provides a product comprising myrrh and rhatany as the sole active ingredients a combined preparation for simultaneous, separate or sequential use in the prevention and/or therapy of viral disease or infections.

The compositions may be formulated in a unit dosage form, eg. with each dosage containing from about 500 to about 1000 mg of the active ingredients.

The precise dosage of the active compounds to be administered and the length of the cause of treatment may, of course, depend on a number of factors including for example, the age and weight of the patient, the specific condition requiring treatment, its severity, and the route of administration. When the composition is administered as a mouthwash however the dosage and rinsing regime is effectively independent of weight, age etc. Also, the known safety of the active ingredients means that the medicament can largely be taken as and when required by the patient and need not normally be strictly monitored.

The administration may be by any suitable method known in the medicinal arts, including for example topical or oral administration or administration by inhalation. In a preferred embodiment the medicament is taken orally.

Alternatively viewed, this aspect of the invention provides a method for the prevention and/or treatment of viral infections or diseases in a human or non-human animal subjects, said method comprising administering to said subject myrrh and rhatany, said myrrh and rhatany being administrable in combination or separately, simultaneously or sequentially.

Viewed from a further aspect, the present invention provides the use of a composition comprising myrrh and rhatany in the prevention and/or treatment of viral infections or diseases in human and non-human animals.

Viewed from yet a further aspect, the present invention provides a pharmaceutical composition comprising myrrh and rhatany as the sole active ingredients together with at least one pharmaceutically acceptable carrier or excipient for use in the prevention and/or treatment of viral infections or diseases in human and non-human animals.

The types of viruses and viral infections suitable for prevention and/or treatment according to the present invention are not limited and include Adenoviruses, Papoviruses, Herpes viruses, Poxviruses, Picornaviruses, Reoviruses, Arboviruses, Myxoviruses, Paramyxoviruses, Leukoviruses and Retroviruses. In a preferred embodiment the viral infections treatable or preventable according to the invention are those caused by members of the Picornaviridae and even more preferably are those caused by Rhiniviruses and Coronaviruses. In the most preferred embodiment the composition comprising myrrh and rhatany as the sole active ingredients is used to prevent and/or treat the common cold, especially the initial stages of the common cold. Advantageously, according to the present invention, the progression or development of the common cold or symptoms of the cold, may be impeded or halted.

In addition to the anti-viral effects of the medicament of the present invention, the combination of myrrh and rhatany possesses antibacterial effects which aid the prevention and cure of bacterial infections which often accompany viral infections. For example secondary infection with Streptococci or Haemophili frequently occurs along with viral infections which cause the common cold and influenza. Thus, the present invention provides a combined antiviral, antibacterial pharmaceutical composition comprising myrrh and rhatany as the sole active ingredients together with at least one pharmaceutically acceptable carrier or excipient.

Remarkably, the Applicant has not only shown the combination of myrrh and rhatany to be effective in the prevention and therapy of viral infections but has shown there to be a synergistic effect between the two active ingredients. In a preferred embodiment therefore the present invention provides an anti-viral composition comprising myrrh and rhatany as synergistically effective sole active agents.

Alternatively viewed, the present invention provides a kit for use in preventing and/or treating viral infections in human and non-human animals comprising:
a) a first container containing myrrh; and
b) a second container containing rhatany.

The invention will now be described in the following non-limiting Examples:

### EXAMPLE 1

A study was conducted using the herbal therapeutic composition of the present invention in the prophylactic treatment of the initial stages of the common cold to illustrate the effectiveness of the composition. The study was conducted over a one-year period with ten people. The herbal composition of the present invention used in the study consisted of a 1:25 dilution of a mixture of equal parts tincture of rhatany and tincture of a myrrh (both tinctures from NAF, Oslo, Norway) in tap water, to form a rinse. Upon first sign of common cold symptoms, such as a sore throat, the test participant gargled once with the rinse until 100 ml was used up. The gargling was repeated after 2 hours if relief was not achieved. With reference to Table 1, those participants who did not develop a cold for 4 days after gargling treatment with the herbal therapeutic composition were scored by a (+), denoting successful treatment. Those test participants who developed anything that the participant defined as a cold were scored with a (-). It is noted that some of the colds experienced were less symptomatic than may have been expected and of short duration.

**TABLE 1**

| Person | Age | Sex | Incidences of Onset of Cold Symptoms | | | | | |
|---|---|---|---|---|---|---|---|---|
| | | | 1 | 2 | 3 | 4 | 5 | 6 |
| AMA | 34 | F | + | + | + | + | + | + |
| MO | 52 | F | + | + | + | | | |
| MBJ | 28 | F | + | + | + | + | | |
| GS | 24 | F | + | + | + | | | |
| AG | 58 | M | - | - | - | | | |
| AP | 34 | F | + | + | - | - | + | - |
| HP | 34 | M | + | + | + | + | + | + |
| EH | 52 | F | + | + | + | + | | |
| JS | 31 | M | - | - | | | | |
| MS | 29 | F | - | - | | | | |

The study involving the ten test participants shows that collectively there were 39 incidences of onset of cold symptoms over a period of twelve months. Using the herbal therapeutic composition of the present invention, as a rinse, the participants showed a successful recession of the cold in 29 of the 39 incidences. The normal spontaneous rate of common cold development in Scandinavia, the geographical area of the study, is approximately 45%. The results of this study show a recovery rate of over 74%, which is significantly better than the normal spontaneous recovery rate of a cold in its early stages of about 30-45%.

### EXAMPLE 2

A mouth wash for gargling may be prepared according to the following formulation:

| | |
|---|---|
| Rhatany tincture | 2.00 ml (400 mg dry weight drug) |
| Myrrh tincture | 2.00 ml (400 mg dry weight drug) |

combined and diluted to 100 ml with tap water. Zinc and other additives such as vitamins or minerals may optionally be added to the mixture.

### EXAMPLE 3

A mouth wash for gargling may be prepared according to the following formulation:

| | |
|---|---|
| Ca(OH)₂ | 5.00 gm |
| Sodium chloride | 8.00 gm |
| Sodium bicarbonate | 2.50 gm |
| Glycerin | 420.00 ml |
| Alcohol | 300.00 ml |
| Menthol | 0.24 gm |
| Thymol | 0.24 gm |
| Methyl salicylate | 0.70 ml |
| Cinnamon oil | 0.50 ml |
| Eucalyptus oil | 1.30 ml |
| Cudbear tincture | 16.00 ml |
| Rhatany tincture | 30.00 ml |
| Myrrh tincture | 30.00 ml |
| Purified talc | 10.00 gm |

## Claims

1. The use of myrrh and rhatany as the sole active ingredients in the preparation of a medicament for the prevention and/or treatment of viral infections or viral diseases in a human or non-human animal subject.

2. A composition comprising myrrh and rhatany as the sole active ingredients together with at least one pharmaceutically acceptable carrier or excipient for use in the prevention and/or treatment of viral infections or viral diseases in a human or non-human animal subject.

3. The use of an alcoholic or hydro-alcoholic solution of myrrh and rhatany in the manufacture of a medicament for the prevention and/or treatment of viral infections or viral diseases in a human or non-human animal subject.

4. A product comprising myrrh and rhatany as the sole active ingredients as a combined preparation for separate, simultaneous or sequential use in the prevention and/or therapy of viral diseases or infections.

5. The use, composition or the product as claimed in any one of claims 1 to 4, wherein said myrrh and rhatany are provided in the form of resins, tinctures, solutions, powders, dried plants or parts of plants.

6. The use, composition or the product as claimed in any one of claims 1 to 5, wherein said viral infection is caused by one or more members of the Picornaviridae.

7. The use, composition or the product as claimed in claim 6, wherein said members of the Picornaviridae are Rhinoviruses and/or Coronaviruses.

8. The use, composition or the product as claimed in claim 7, wherein said viral infection is the common cold.

9. The use, composition or the product as claimed in any one of claims 1 to 8, wherein said myrrh and rhatany are provided in the form of alcoholic tinctures.

10. The use, composition or the product as claimed in claim 9, wherein the rhatany or myrrh in said alcoholic tinctures is approximately 20% w/w.

11. The use, composition or the product as claimed in either of claims 9 or claim 10, wherein said alcoholic tinctures are diluted approximately 1:10 to 1:100 to provide a composition suitable for administration.

12. The use, composition or the product as claimed in claim 11, wherein said tinctures are diluted approximately 1:25 with water to provide the composition for administration.

13. The use, composition or the product as claimed in any one of claims 9 to 12, wherein said tinctures of myrrh and rhatany are combined in equal amounts with or without dilution to provide a composition for administration.

14. The use, composition or the product as claimed in any one of claims 1 to 13, wherein said myrrh and rhatany are in a form suitable for administration topically or orally.

15. The use, composition or the product as claimed in claim 14, wherein said oral administration is by means of a mouthwash or gargle.

16. The use as claimed in claim 3, wherein vitamins or minerals are added to the medicament.

## Patentansprüche

1. Verwendung von Myrrhe und Ratanhia als die einzigen aktiven Inhaltsstoffe bei der Zubereitung eines Medikaments für die Vorbeugung und/oder Behandlung von Virusinfektionen oder Viruserkrankungen bei einem Menschen oder nichtmenschlichen Tier.

2. Zusammensetzung, die Myrrhe und Ratanhia als die einzigen Inhaltsstoffe zusammen mit mindestens einem pharmazeutisch zulässigen bzw. brauchbaren Träger oder Grundstoff aufweist, zur Verwendung bei der Vorbeugung und/oder Behandlung von Virusinfektionen oder Viruserkrankungen bei einem Menschen oder einem nichtmenschlichen Tier.

3. Verwendung einer alkoholischen oder wäßrigalkoholischen Lösung von Myrrhe und Ratanhia bei der Herstellung eines Medikaments für die Vorbeugung und/oder die Behandlung von Virusinfektionen oder Viruserkrankungen bei einem Menschen oder einem nichtmenschlichen Tier.

4. Erzeugnis, das Myrrhe oder Ratanhia als die einzigen aktiven Inhaltsstoffe als eine Kombinationszubereitung aufweist, für eine getrennte, gleichzeitige oder schrittweise Verwendung bei der Vorbeugung und/oder der Therapie von Viruserkrankungen oder -infektionen.

5. Verwendung, Zusammensetzung oder Erzeugnis nach einem der Ansprüche 1 bis 4, wobei die Myrrhe und Ratanhia in der Form von Harzen, Tinkturen, Lösungen, Pulvern, getrockneten Pflanzen oder Pflanzenteilen bereitgestellt sind.

6. Verwendung, Zusammensetzung oder Erzeugnis nach einem der Ansprüche 1 bis 5, wobei die Virusinfektion durch eine oder mehrere Mitglieder der Familie Picornaviridae verursacht ist.

7. Verwendung, Zusammensetzung oder Erzeugnis nach Anspruch 6, wobei die Mitglieder der Familie Picornaviridae Rhinoviren und/oder Coronaviren sind.

8. Verwendung, Zusammensetzung oder Erzeugnis nach Anspruch 7, wobei die Virusinfektion eine herkömmliche Erkältung ist.

9. Verwendung, Zusammensetzung oder Erzeugnis nach einem der Ansprüche 1 bis 8, wobei die Myrrhe und Ratanhia in der Form von alkoholischen Tinkturen vorgesehen ist.

10. Verwendung, Zusammensetzung oder Erzeugnis nach Anspruch 9, wobei die Ratanhia oder Myrrhe in der alkoholischen Tinktur etwa 20 % w/w beträgt.

11. Verwendung, Zusammensetzung oder Erzeugnis nach einem der Ansprüche 9 oder 10, wobei die alkoholischen Tinkturen etwa 1:10 bis 1:100 verdünnt sind, um eine für eine Verabreichung geeignete Zusammensetzung bereitzustellen.

12. Verwendung, Zusammensetzung oder Erzeugnis nach Anspruch 11, wobei die Tinkturen mit Wasser etwa 1:25 verdünnt sind, um die Zusammensetzung für eine Verabreichung bereitzustellen.

13. Verwendung, Zusammensetzung oder Erzeugnis nach einem der Ansprüche 9 bis 12, wobei die Tinkturen von Myrrhe oder Ratanhia in gleichen Mengen mit oder ohne Verdünnung kombiniert sind, um eine Zusammensetzung für eine Verabreichung bereitzustellen.

14. Verwendung, Zusammensetzung oder Erzeugnis nach einem der Ansprüche 1 bis 13, wobei die Myrrhe und Ratanhia in einer Form vorliegen, die für eine örtliche oder orale Verabreichung geeignet ist.

15. Verwendung, Zusammensetzung oder Erzeugnis nach Anspruch 14, wobei die orale Verabreichung mittels Mundspülen oder Gurgeln erfolgt.

16. Verwendung nach Anspruch 3, wobei dem Medikament Vitamine oder Mineralien zugegeben sind.

## Revendications

1. Utilisation de myrrhe et de ratanhia en tant que principes actifs uniques dans la préparation d'un médicament pour la prévention et/ou le traitement d'infections virales ou de maladies virales chez un être humain ou un animal.

2. Composition comprenant de la myrrhe et du ratanhia en tant que principes actifs uniques ainsi qu'au moins un véhicule ou excipient pharmaceutiquement acceptable pour une utilisation dans la prévention et/ou le traitement d'infections virales ou de maladies virales chez un être humain ou un animal.

3. Utilisation d'une solution alcoolique ou hydro-alcoolique de myrrhe et de ratanhia dans la fabrication d'un médicament pour la prévention et/ou le traitement d'infections virales ou de maladies virales chez un être humain ou un animal.

4. Produit comprenant de la myrrhe et du ratanhia en tant que principes actifs uniques sous forme de préparation combinée pour une utilisation séparée, simultanée ou séquentielle dans la prévention et/ou le traitement de maladies ou infections virales.

5. Utilisation, composition ou produit selon l'une quelconque des revendications 1 à 4, dans lesquels lesdits myrrhe et ratanhia sont fournis sous forme de résines, teintures, solutions, poudres, plantes séchées ou parties de plantes.

6. Utilisation, composition ou produit selon l'une quelconque des revendications 1 à 5, dans lesquels ladite infection virale est causée par un ou plusieurs membres des Picornaviridés.

7. Utilisation, composition ou produit selon la revendication 6, dans lesquels lesdits membres des Picornaviridés sont des rhinovirus et/ou des coronavirus.

8. Utilisation, composition ou produit selon la revendication 7, dans lesquels ladite infection virale est le rhume banal.

9. Utilisation, composition ou produit selon l'une quelconque des revendications 1 à 8, dans lesquels lesdits myrrhe et ratanhia sont fournis sous forme de teintures alcooliques.

10. Utilisation, composition ou produit selon la revendication 9, dans lesquels le ratanhia ou la myrrhe représente approximativement 20% m/m desdites teintures alcooliques.

11. Utilisation, composition ou produit selon la revendication 9 ou la revendication 10, dans lesquels lesdites teintures alcooliques sont diluées approximativement de 1/10 à 1/100 pour fournir une composition convenant à une administration.

12. Utilisation, composition ou produit selon la revendication 11, dans lesquels lesdites teintures sont diluées approximativement à 1/25 avec de l'eau pour fournir la composition à administrer.

13. Utilisation, composition ou produit selon l'une quelconque des revendications 9 à 12, dans lesquels lesdites teintures de myrrhe et de ratanhia sont combinées en quantités égales avec ou sans dilution pour fournir une composition à administrer.

14. Utilisation, composition ou produit selon l'une quelconque des revendications 1 à 13, dans lesquels lesdits myrrhe et ratanhia sont sous une forme convenant à une administration locale ou orale.

15. Utilisation, composition ou produit selon la revendication 14, dans lesquels ladite administration orale se fait au moyen d'un collutoire ou d'un gargarisme.

16. Utilisation selon la revendication 3, dans laquelle des vitamines ou minéraux sont ajoutés au médicament.
